# FASCICULE DE BREVET EUROPEEN

(11) **EP 3 570 789 B1**
(45) Date de publication et mention de la délivrance du brevet: **25.06.2025**
(21) Numéro de dépôt: 18702781.8
(22) Date de dépôt: 23.01.2018
(51) Int. Cl.: A61F 2/46, A61F 2/30, A61B 17/15

(54) **ANCILLAIRE ET PROCDE DE FABRICATION D'UN ANCILLAIRE**
HILFSVORRICHTUNG UND VERFAHREN ZUR HERSTELLUNG EINER HILFSVORRICHTUNG
ANCILLARY DEVICE AND METHOD FOR MANUFACTURING AN ANCILLARY DEVICE

(30) Priorité: 23.01.2017 FR 1750543
(43) Date de publication de la demande: 27.11.2019
(73) Titulaire: 3D Medical, 94440 Marolles-En-Brie (FR)
(72) Inventeur: GEMON, Jean-Pierre, 33000 Bordeaux (FR); NUTTENS, Vincent, 91800 Brunoy (FR)
(74) Mandataire: Plasseraud IP
(86) Numéro de dépôt international: PCT/FR2018/050152
(87) Numéro de publication internationale: WO 2018/134545

(56) Documents cités:
- WO-A1-2016/074733
- FR-A1- 2 938 178
- FR-A1- 3 024 027

## Description

La présente invention concerne le domaine de la chirurgie orthopédique, et plus particulièrement un ancillaire de guidage et son procédé de fabrication. La présente invention a trait également à un ancillaire de guidage pour son utilisation en chirurgie.

La chirurgie orthopédique traite les maladies, les traumatismes (fracture, rupture, hématome), et les déformations de l'appareil locomoteur : os, articulations, ligaments, tendons et muscles. Autrefois limitée au seul traitement des fractures, elle se développe désormais dans d'autres domaines : prothèses articulaires ou chirurgie endoscopique.

Ce domaine est soumis à de nombreuses avancées technologiques du fait de la quantité importante d'instruments nécessaires à sa réalisation, et notamment concernant les ancillaires, se définissant comme un instrument destiné à guider les outils nécessaires aux actes chirurgicaux.

Le développement des technologies de communication, des outils informatiques et des robots a énormément progressé, et notamment en chirurgie orthopédique. Toute la difficulté est de reproduire dans un environnement en trois dimensions une planification faite en deux dimensions. La chirurgie robotique a ainsi été introduite et se présente comme un outil dont le geste est fait en tout ou partie par robot. Les contraintes liées à l'usage de robots actifs ou semi-actifs au bloc opératoire peuvent être associées à la réticence des chirurgiens à laisser faire un geste chirurgical à une machine, mais également à des coûts d'investissement et problématiques d'entretien importants.

Un système passif s'est développé plus largement dans les années suivantes. La navigation consiste en un système d'orientation dans l'espace mis en rapport avec l'anatomie du patient. Ce système de navigation correspond à un outil de mesure dont les valeurs sont retranscrites dans une unité centrale avec une interface pour le chirurgien. La navigation permet une augmentation de la précision et de la reproductibilité du geste chirurgical. En préopératoire, il est possible de planifier l'intervention dans l'ordinateur du système de navigation avec une imagerie chargée dans l'ordinateur. L'interface montre une représentation de l'anatomie du site opératoire avec la possibilité de combiner les planches d'imagerie chargées, des graphiques ou les mesures effectuées en temps réel et avec une grande précision. Pendant l'intervention, elle permet la représentation des parties anatomiques non visibles et le suivi des instruments ou de l'alignement des implants en temps réel.

La navigation a permis une amélioration de la précision dans l'implantation de prothèses et dans d'autres domaines en orthopédie (chirurgie de la scoliose, ostéotomies correctrices, traumatologie). Néanmoins, la durée d'intervention est rallongée par l'utilisation de la navigation. L'encombrement du bloc opératoire est également important. Enfin, le coût d'investissement initial et d'entretien n'est pas négligeable pour des structures de petites et moyennes tailles.

Malgré l'amélioration de la précision obtenue grâce aux robots et à la navigation à distance, l'augmentation de la quantité d'instruments et du nombre d'étapes chirurgicales, responsable d'un allongement de la durée de la procédure ne sont pas en faveur d'une rentabilité économique.

Les guides personnalisés sont apparus au milieu des années 90. A partir d'une imagerie préopératoire volumétrique, un logiciel spécifique définit des points de repères pour positionner sur le patient un guide dit guide personnalisé qui permettra de réaliser la planification enregistrée sur l'ordinateur sans modifications ni ajustement pendant l'intervention.

Initialement un bloc en plastique était découpé sur mesure afin de suivre les reliefs exacts des os du patient modélisé en 3D. Lesdits blocs peuvent être utilisés comme interface entre l'os et l'ancillaire standard ou comme guide pour le positionnement d'une mèche, d'une broche ou d'une scie.

Par la suite, la technologie a pris son essor grâce aux améliorations techniques du prototypage rapide permettant les impressions en 3D rapides avec une précision accrue, une plus grande variété de matériaux disponibles et une diminution des coûts.

En 2006, Hafez et al. décrivaient la procédure moderne de réalisation d'une chirurgie avec instrumentation personnalisée pour la prothèse totale de genou :
- Réalisation d'une imagerie par tomodensitométrie (TDM) ou Imagerie par Résonnance Magnétique (IRM) du membre inférieur ciblée sur le genou, la hanche et la cheville.
- Les données sont transférées de façon sécurisée vers un centre spécialisé pour reconstruction en 3D après segmentation des coupes.
- Une planification préopératoire en 3D (taille des implants, positionnement des implants, coupes osseuse nécessaires, repères osseux pour l'assise du guide) est réalisée par un ingénieur grâce à la superposition des implants sur l'os et avec l'alignement du membre inférieur comme référence.
- Le contrôle final revient au chirurgien qui peut modifier l'ensemble des données paramétrées sur l'alignement et le positionnement des implants. Il valide la planification ainsi que les repères adaptés à la voie d'abord chirurgicale pour le positionnement du guide.
- Le guide de coupe est fabriqué selon un procédé de prototypage rapide.

Les guides personnalisés ont ainsi été développés en orthopédie pour améliorer la précision des coupes osseuses et réduire le temps lié à ce geste. Ils permettent d'appliquer durant l'intervention une planification réalisée sur des images reconstruites en 3D. C'est dans la chirurgie du genou et notamment dans l'arthroplastie totale du genou qu'ils ont été le plus développés. Il existe d'autres applications au genou, à la hanche, à l'épaule ou à la cheville principalement dans le cadre des arthroplasties mais aussi au rachis pour le positionnement des vis pédiculaires, dans le cadre des résections tumorales pour guider les coupes osseuses sans pénétrer la lésion et pour les ostéotomies correctrices de membres.

Les guides sont prévus pour le positionnement des broches ou pour diriger la coupe. Les guides positionneurs de broche permettent d'introduire les broches qui vont guider le positionnement du guide de coupe standard. Le retrait du guide personnalisé pour disposer le guide de coupe standard augmente le risque d'imprécision lié à la fragilité de l'os et à la modification de la position des broches durant la manipulation.

Les guides de coupes sont les plus représentés chez les fabricants, positionnés sur l'os grâce aux repères d'appui osseux spécifiques, fixés par 2 ou 3 broches pour augmenter leur stabilité, ils sont pourvus d'une fente par laquelle passe exactement la scie qui sert à découper l'os. Le guide fémoral est fixé à la partie distale du fémur, il permet la réalisation de la coupe distale ainsi que le marquage de repères pour le positionnement du guide dit « 4 en 1 », permettant de réaliser les coupes antérieures, postérieures ainsi que les chanfreins antérieurs et postérieurs. Le guide tibial est fixé à la partie antérieure et proximale du tibia, il permet de réaliser la coupe proximale du tibia (Thèse pour le diplôme d'état de docteur en médecine : ancillaire de coupe sur mesure : application en orthopédie et résultats dans la prothèse du genou).

Enfin, les ancillaires de coupe par sciage connus de l'état de la technique sont étudiés et conçus de manière à implanter des prothèses standards.

La technique chirurgicale de pose d'une prothèse totale de genou est une technique invasive et entraine un traumatisme de la structure anatomique. Les voies d'abord de l'articulation du genou nécessitent la luxation de l'articulation. Par ailleurs, il peut être nécessaire de sacrifier le ligament croisé antérieur et/ou ligament croisé postérieur.

De plus les guides de coupe personnalisée sont conçus de sorte à s'adapter à chaque surface osseuse afin de pratiquer une coupe de l'os, pouvant entrainer un décalage ainsi qu'un déséquilibre de l'articulation lors de la coupe osseuse.

Il existe ainsi un besoin d'un ancillaire apte à coopérer avec plusieurs surfaces osseuses, permettant une meilleure stabilité et un meilleur ancrage de l'ancillaire. Il existe également un besoin pour un ancillaire n'imposant pas une luxation des articulations, ni de sacrifice des ligaments y afférents, et respectant les structures anatomiques. Enfin, il existe un besoin pour un ancillaire sur-mesure, permettant l'implantation d'une prothèse sur mesure.

Le document D1(FR3024027) décrit un dispositif chirurgical comprend un organe de stabilisation intra articulaire, destiné à être positionné entre les deux os de l'articulation, et deux blocs de guidage fixés à l'organe de stabilisation et de part et d'autre d'un plan défini par ledit organe de stabilisation, lesdits blocs de guidage comprennent des moyens de fixation à l'os et présentent chacun au moins une fente pour l'engagement et le guidage d'un instrument destiné à réaliser des opérations de coupes de parties des os.

Le document D2 (WO20216/074733) décrit une trousse chirurgicale permettant de réparer des surfaces articulaires dans l'articulation tibio-tarsienne, comprenant : un implant chirurgical ayant un capuchon avec une surface externe qui se conforme à une surface du dôme de l'astragale et une surface interne qui présente une cheville d'ancrage d'implant centrale qui s'étend perpendiculairement à partir de ladite surface interne, ue enveloppe tubulaire creuse permettant de réaliser un préperçage correct pour l'implantation de l'implant d'articulation chirurgical dans le dôme de l'astragale, un guide pour scie qui se conforme et qui peut être fixé à la partie inférieure du tibia et qui fournit des surfaces de guidage de scie dans au moins un plan, permettant de réaliser une ostéotomie d'une partie inférieure d'un tibia afin de faire apparaître le dôme de l'astragale.

### OBJET DE L'INVENTION

La présente invention vise à pallier les inconvénients de l'art antérieur et à répondre aux contraintes ci-dessus énoncées en proposant un ancillaire de guidage destiné à coopérer avec au moins deux surfaces osseuses.

La présente invention est définie dans la revendication 1 qui divulgue un ancillaire de guidage et également la revendication 13 qui a pour objet un procédé de fabrication de l' ancillaire de guidage , tandis que les modes de réalisation préférés sont décrits dans les revendications dépendantes.

Les méthodes associées sont également décrites ici pour faciliter la compréhension de l'invention. Ces méthodes ne font pas partie de l'invention revendiquée.

Elle concerne également un ancillaire de guidage pour son utilisation en chirurgie orthopédique.

Elle concerne encore un ensemble comprenant un ancillaire de guidage selon la présente invention et au moins un dispositif médical.

### BREVE DESCRIPTION DE L'INVENTION

L'invention concerne ainsi un ancillaire de guidage destiné à coopérer avec au moins deux surfaces osseuses caractérisé en ce qu'il comprend :
- Pour chaque surface osseuse, une surface d'appui destinée à épouser au moins en partie une portion de la surface osseuse correspondante ; ladite surface d'appui délimite en outre au moins une zone de travail sur ladite surface osseuse lorsqu'elle est en contact avec ladite surface osseuse;
- lesdites surfaces d'appui étant configurées pour maintenir en position ledit ancillaire sur les surfaces osseuses correspondantes, et
- Au moins un moyen de guidage apte à recevoir au moins un dispositif médical, ledit au moins un moyen de guidage guidant le dispositif médical vers ladite zone de travail sur la surface osseuse correspondante.

On entend par surface osseuse au sens de l'invention, l'extrémité d'un os ou la surface externe d'une partie d'un os.

On entend par surface d'appui au sens de l'invention, la partie de l'ancillaire destinée à venir en contact avec au moins une partie de la surface osseuse correspondante.

L'ancillaire de guidage au sens de la présente invention est défini et réalisé après notamment acquisition d'une ou plusieurs images par imagerie par tomodensitométrie (Scanner) et/ou Imagerie par Résonnance Magnétique (IRM) ou tout autre procédé d'acquisition d'images de au moins deux surfaces osseuses et représentation en 3D des images ainsi obtenues, et détermination des modifications à apporter sur au moins l'une des surfaces osseuses. Ainsi, l'ancillaire, au travers de chacune de ses surfaces d'appui, épouse au moins en partie une portion de la surface osseuse correspondante et permet donc un référencement précis de la surface de au moins une partie de la surface osseuse. L'ancillaire sera déterminé par notamment et au moins la détermination d'au moins une modification à apporter sur au moins une des surfaces osseuses. Ainsi, et contrairement à un ancillaire personnalisé, permettant la mise en place d'implant standard, l'ancillaire selon la présente invention est un ancillaire sur-mesure s'adaptant non seulement aux caractéristiques telles que la forme, les dimensions, des surfaces osseuses avec lesquelles il est destiné à être mis en œuvre mais surtout aux modifications et/ou interventions à apporter sur au moins l'une des surfaces osseuses de chaque patient. Cet ancillaire sur-mesure est donc adapté et personnalisé pour chaque intervention sur chaque patient et ne saurait donc être réutilisable.

Avantageusement, les surfaces d'appuis de l'ancillaire permettent de maintenir et sécuriser en position celui-ci et ainsi réduire son risque de déplacement. L'ancillaire selon la présente invention permet ainsi un meilleur alignement dudit ancillaire avec au moins deux surfaces osseuses durant l'acte chirurgical. Outre cette fonction d'appui améliorée, chaque surface d'appui dudit ancillaire de guidage permet de définir une zone de travail.

On entend par zone de travail au sens de la présente invention, la zone de la surface osseuse délimitée par la surface d'appui de l'ancillaire de guidage selon la présente invention.

Les surfaces d'appui de l'ancillaire de guidage délimite chacune une surface de travail et les moyens de guidage dirigeront les dispositifs médicaux vers la zone de travail de chacune des surfaces osseuses correspondantes.

On entend par dispositif médical au sens de la présente invention, tout instrument, appareil, équipement, matière utilisé seul ou en association pour son utilisation dans le diagnostic, la prévention et/ou le traitement d'une maladie, l'étude, le remplacement ou la modification de l'anatomie ou d'un processus physiologique. On citera à titre purement illustratif, un instrument de visée, un instrument de fraisage, un instrument de perçage, un instrument de cimentoplastie, un instrument de vissage, un instrument de destruction cellulaire, un instrument d'excitation cellulaire, un instrument de biopsie, un instrument de sciage, une sonde, un cathéter, un ballonnet, un stent, et tout instrument prenant référence sur l'os pour une intervention ou mesure ou positionnement.

On entend par moyen de guidage au sens de la présente invention, tout moyen permettant de guider le déplacement d'un dispositif médical vers au moins une zone de travail prédéfinie. On citera pour exemple un élément tubulaire, des fentes orientées, un chemin de déplacement ou des combinaisons de ces éléments.

En outre, l'ancillaire de guidage au sens de la présente invention permet de positionner l'ancillaire sur au moins deux surfaces osseuses afin de guider au moins un dispositif médical. Les structures anatomiques sont ainsi avantageusement préservées. De manière avantageuse, cet ancillaire n'implique donc pas de luxation de l'articulation durant sa mise en place et d'éventuels sacrifices de ligaments.

Dans différents modes de réalisation particuliers de l'ancillaire de guidage, chacun ayant ses avantages particuliers et susceptibles de nombreuses combinaisons techniques possibles :
- L'ancillaire de guidage est d'un seul tenant.

L'ancillaire de guidage d'un seul tenant selon la présente invention permet donc un contact intime et stable avec au moins deux surfaces osseuses, imposant ainsi une orientation unique et offrant une stabilité maximale à l'ancillaire selon la présente invention.
- L'ancillaire de guidage comporte au moins deux parties d'ancillaire, chacune des parties d'ancillaire étant destinée à coopérer avec au moins une surface osseuse distincte.

Typiquement, et dans ce mode de réalisation, l'ancillaire comprend au moins un moyen d'assemblage permettant de lier lesdites au moins deux parties d'ancillaire.

Typiquement, le moyen d'assemblage ou liaison est rigide ou non.
- Chaque partie d'ancillaire comporte une portion d'assemblage femelle et/ou une portion d'assemblage mâle, deux parties d'ancillaire destinées à être immédiatement contigües lorsque ledit ancillaire est assemblé, présentant une zone d'assemblage femelle et une zone d'assemblage mâle destinées à coopérer entre elles pour assurer l'assemblage desdites deux parties d'ancillaire, au moins certaines desdites parties d'ancillaire comprenant au moins un moyen de guidage, lesdits moyens de guidage étant distincts ou au moins certaines desdites parties d'ancillaire comprenant une portion de moyen de guidage, lesdites portions de moyen de guidage étant distinctes et destinées à former un moyen de guidage lorsque lesdites parties d'ancillaires sont assemblées entre elles.

A titre purement illustratif, et dans le cas où l'ancillaire est constitué de deux parties d'ancillaire, une première partie d'ancillaire comporte à son extrémité opposée à celle destinée à prendre appui sur la surface osseuse correspondante, une portion d'assemblage mâle, laquelle est destinée à coopérer avec une portion d'assemblage femelle placée à une extrémité correspondante de la seconde partie d'ancillaire afin d'assurer un assemblage stable desdites première et seconde parties d'ancillaire. Bien entendu, et de manière avantageuse, ces portions d'assemblage autorisent leur séparation après assemblage de sorte que celui-ci peut être temporaire. L'assemblage peut ainsi être du type à clipsage, par emboitement, ...

A titre purement illustratif, le moyen d'assemblage est choisi parmi une charnière, une articulation, un moyen de guidage permettant un mouvement de translation, ou de rotation.

Le moyen d'assemblage pourra être une surface de référence permettant le déplacement par glissement, une butée.
- Typiquement, la surface de référence permettant le déplacement par glissement pourra être choisie parmi un rail ou un chemin de déplacement. Lesdites parties d'ancillaires comprennent, pour chaque zone d'assemblage mâle ou femelle, au moins un moyen détrompeur pour empêcher l'assemblage de parties d'ancillaire non destinées à être immédiatement adjacentes lorsque l'ancillaire est assemblé.
- Les parties d'ancillaire sont configurées pour définir au moins 2 axes de guidage d'un ou plusieurs dispositifs médicaux différents lorsqu'elles sont assemblées entre elles.

Dans un mode de réalisation préféré, les parties d'ancillaire sont configurées pour définir 4 ou 5 axes de guidage d'un ou plusieurs dispositifs médicaux différents lorsqu'elles sont assemblées entre elles.
- Chaque partie de l'ancillaire comprend pour chaque surface osseuse une surface d'appui destinée à épouser au moins en partie une portion de ladite surface osseuse correspondante, ladite surface d'appui délimitant une zone de travail sur ladite surface osseuse, chaque partie comprenant au moins deux moyens de guidage, lesdits au moins deux moyens de guidage définissant des axes de travail distincts, pour guider le déplacement d'au moins un dispositif médical le long desdits axes de travail sur la surface osseuse correspondante.
- L'ancillaire de guidage est configuré pour coopérer avec deux surfaces osseuses.

Préférentiellement, les deux surfaces osseuses sont la surface osseuse du tibia et la surface osseuse du fémur, préférentiellement, la surface osseuse de l'extrémité distale du tibia et la surface osseuse de l'extrémité proximale du fémur.

Préférentiellement encore, chaque surface d'appui est une empreinte de la portion de la surface osseuse de l'extrémité distale fémorale et de l'extrémité proximale tibiale, lorsque le genou est en position anatomique de référence, et n'épouse pas les surfaces osseuses de la cavité intra-articulaire.

Ainsi et de manière très avantageuse, lorsque l'ancillaire selon la présente invention est configuré pour coopérer avec deux surfaces osseuses, telles que la surface osseuse du tibia et la surface osseuse du fémur, l'ancillaire n'impose pas de luxation de l'articulation lors de sa mise en place sur les deux surfaces osseuses, ni de sacrifier l'un quelconque des ligaments de l'articulation. L'ancillaire selon la présente invention coopère avec les surfaces osseuses sans s'insérer dans la cavité intra-articulaire. Cet ancillaire sur-mesure s'adapte à la morphologie du patient et permet d'implanter une prothèse sur mesure en fonction de la pathologie et/ou du dysfonctionnement.

Avantageusement, chaque partie de l'ancillaire comprend pour la surface tibiale et la surface fémorale, une surface d'appui destinée à épouser au moins en partie une portion de la surface osseuse correspondante, ladite surface d'appui délimitant une zone de travail sur ladite surface osseuse, chaque partie comprenant au moins deux moyens de guidage, lesdits au moins deux moyens de guidage définissant des axes de travail distincts, pour guider le déplacement d'au moins un dispositif médical le long desdits axes de travail sur la surface osseuse correspondante. Cette configuration permet ainsi d'éviter une luxation de l'articulation et de préserver l'anatomie et les structures tissulaires et musculaires du patient.

Dans un autre mode de réalisation, l'ancillaire de guidage est configuré pour coopérer avec trois surfaces osseuses.
- Ledit ou au moins un desdits moyens de guidage est configuré pour définir une butée pour le déplacement dudit dispositif de guidage correspondant.

Par exemple pour limiter la course du dispositif médical de guidage le long de l'axe de déplacement défini par ledit moyen de guidage - A titre d'exemple, la dimension axiale d1 du moyen de guidage limite la course d'un outil de dimension axiale d2 supérieur à d1 tel que d2-d1 = profondeur de pénétration du dispositif médical dans la zone lésée à réparer.
- Le au moins un moyen de guidage est choisi parmi un élément tubulaire, des fentes orientées, un chemin de déplacement, un élément allongé creux ou des combinaisons de ces éléments.

Au sens de la présente invention, un chemin de déplacement ou chemin de guidage s'entend d'un objet présentant une surface en relief, continue ou discontinue, permettant de guider le déplacement d'au moins un dispositif médical vers au moins une zone de travail.

A titre purement illustratif, cet objet peut présenter des rainures, débouchant à chacune de leurs extrémités, pour permettre le guidage et le passage au travers de chacune d'entre elles d'au moins un dispositif médical. Ces rainures peuvent présenter des formes différentes pour guider différemment ledit au moins un dispositif médical correspondant en fonction de la nature de la modification locale à apporter à la surface osseuse correspondante. On entend par fente orientée, un élément présentant une ouverture permettant de laisser passer un dispositif médical, et ce afin de guider ledit dispositif médical vers la zone de travail à la surface de l'extrémité osseuse.

On entend par élément tubulaire, un élément rectiligne, percé en son centre d'une lumière également rectiligne parallèle à la dimension principale de l'élément tubulaire.

Dans un autre mode de réalisation, l'ancillaire de guidage comprend plusieurs moyens de guidage, lesdits moyens de guidage étant une combinaison d'au moins deux moyens de guidage choisis parmi un élément tubulaire, un chemin de guidage, une fente orientée.

On entend par élément allongé creux un élément comprenant un canal intérieur ou canal de guidage, apte à recevoir un dispositif médical, la surface intérieure dudit canal intérieur étant un élément de guidage permettant de guider et de déplacer l'outil vers la zone de travail à la surface de l'extrémité osseuse.
- chaque élément allongé creux comporte un canal pour le passage d'au moins un dispositif médical au travers dudit élément allongé creux correspondant, la paroi intérieure dudit élément allongé creux délimitant ledit canal définissant une surface de déplacement dudit dispositif médical contre au moins une partie de laquelle est destinée à être déplacé ledit dispositif médical afin de délimiter l'excursion dudit dispositif médical dans ladite zone de travail de ladite surface osseuse correspondante.

Avantageusement, la surface de déplacement du dispositif médical est configurée de sorte à ce que le déplacement de l'outil sur cette surface détermine le pourtour de la modification locale à apporter sur la surface osseuse.
- l'élément allongé creux comprend une extrémité libre, la paroi intérieure d'au moins ladite extrémité libre est évasée ou de forme oblongue, ou de forme tubulaire aplatie, de section droite de forme elliptique, polygonale telle que rectangulaire, carrée.

On entend par extrémité libre de l'élément allongé creux, la partie la plus proche de la surface osseuse.

A titre illustratif, lorsque la paroi intérieure de ladite extrémité libre est oblongue, la surface intérieure dudit canal intérieur permet de guider le dispositif médical vers la zone de travail à la surface de l'extrémité osseuse ainsi que le déplacement latéral du dispositif médical sur la zone de travail à la surface de l'extrémité osseuse.

Le moyen de guidage et la forme du canal intérieur seront ainsi déterminés en fonction de l'action à réaliser dans la zone de travail à la surface de l'extrémité osseuse.

Selon ce mode de réalisation, le diamètre d'ouverture du canal intérieur sera déterminé en fonction de l'action à réaliser.

Dans un autre mode de réalisation, lorsque l'ancillaire selon la présente invention comprend au moins deux moyens de guidage comprenant un canal intérieur, les moyens de guidage pourront être soit identiques, soit différents, le diamètre et/ou la forme du canal intérieur de chacun des éléments de guidage pourra/pourront être distinct(s), en fonction de l'action à réaliser.

Dans un mode de réalisation préféré, tous les éléments de guidage sont des éléments tubulaires.
- chaque élément tubulaire est fonction de la surface osseuse correspondante du patient pour recevoir un dispositif médical dont au moins le diamètre est lié aux dimensions de ladite surface osseuse.
- l'élément tubulaire comporte une lumière définissant un axe longitudinal pour le déplacement d'au moins un dispositif médical le long dudit axe.

Les dimensions de l'élément tubulaire sont telles que ledit élément tubulaire est apte et destiné à recevoir un dispositif médical et à guider ledit dispositif médical vers la zone de travail à la surface de la surface osseuse, ledit dispositif médical étant inséré dans la lumière dudit élément tubulaire.

Dans un mode de réalisation, les éléments tubulaires sont identiques.

Dans un autre mode de réalisation, le diamètre de la lumière des éléments tubulaire peut être différent en fonction du dispositif médical devant être inséré dans la lumière dudit élément tubulaire.
- L'ancillaire de guidage comprend au moins deux moyens de guidage.

Dans un mode de réalisation tout particulièrement préféré, l'ancillaire de guidage comprend huit moyens de guidage, lesdits huit moyens de guidage étant des éléments tubulaires.
- Le moyen de guidage définit au moins un axe de travail pour guider un dispositif médical sur ladite au moins une zone de travail sur la surface osseuse afin de modifier localement au moins une portion de ladite surface osseuse correspondante dans ladite au moins une zone de travail.
- L'élément tubulaire comprend un axe principal, cet axe principal est confondu avec l'axe de travail, l'axe de travail dudit élément de guidage étant dirigé selon un axe tangent ou sensiblement tangent en un point de la zone de travail.
- Le moyen de guidage est configuré de sorte à diriger le dispositif médical dans une partie d'au moins une des zones de travail, ladite partie étant séparée d'une zone sensible.

On entend par zone sensible, notamment une zone innervée, une zone vascularisée, une zone médullaire. Ainsi, on entend par zone sensible, le paquet vasculo-nerveux et la moelle, ladite zone sensible étant distincte d'une zone osseuse.

Avantageusement, l'ancillaire selon la présente invention permet d'appuyer le geste chirurgical et le guider afin d'éviter l'une quelconque des zones sensibles.
- L'ancillaire de guidage est constitué d'un matériau thermofusible et biocompatible.

On entend par matériau thermofusible au sens de la présente invention un matériau devenant fluide sous l'effet de la chaleur. De manière avantageuse, le matériau de l'ancillaire au sens de la présente invention est biocompatible, c'est-à-dire qu'il a la capacité de ne pas interférer et de ne pas dégrader le milieu biologique dans lequel il est utilisé. On citera par exemple les métaux et alliages métalliques tels que les aciers inoxydables, l'inox, et plus particulièrement l'inox 316L ou l'inox 17-4PH, le titane et les alliages de titane tels que le Titane de grade 1, de grade 2, de grade 4, de grade 5, de grade 23, les céramiques telles que l'alumine et la zircone, des polymères tels que des copolymères d'acide lactique et glycolique, les polyanhydrides, des polyaminoacides, des polyamides, des matériaux d'origine naturelle tels que la chitine, les fucanes, la cellulose, le corail, le collagène.

Préférentiellement, le matériau de l'ancillaire est le polyamide 12 ou le titane. Typiquement, l'ancillaire selon la présente invention pourra être utilisée durant toute opération chirurgicale.

A titre purement illustratif, on citera l'ostéosynthèse, l'arthrodèse, l'arthroplastie, la cimentoplastie, l'ostéotomie, la neurochirurgie et la kyphoplastie.

Alternativement, l'ancillaire pourra être utilisé en système *ex vivo* d'entrainement chirurgical (modèle de formation, entrainement avant chirurgie, validation du mode opératoire). Il pourra également être utilisé sur des modèles cadavériques en vue de l'obtention de preuves de concept.

Un autre aspect de l'invention est un procédé de fabrication d'un ancillaire de guidage.

Ainsi et selon l'invention, le procédé de fabrication de l'ancillaire de guidage comprend les étapes suivantes :
i. Acquisition d'une ou plusieurs images par tomodensitométrie (Scanner, Rayon X) et/ou Imagerie par Résonnance Magnétique, de au moins deux surfaces osseuses ;
ii. Représentation en 3D de l'imagerie des au moins deux surfaces osseuses acquises à l'étape i. ;
iii. Détermination d'au moins un axe de travail pour modifier localement au moins une portion d'au moins une desdites surfaces osseuses ;
iv. Détermination d'au moins une surface d'appui sur lesdites surfaces osseuses pour le maintien en position de l'ancillaire, au moins une desdites surfaces d'appui délimitant au moins une zone de travail dans laquelle on cherche à modifier localement ladite au moins une portion d'au moins une surface osseuse ;
v. Positionnement d'au moins un moyen de guidage, ledit au moins un moyen de guidage étant configuré de manière à guider au moins un dispositif médical le long dudit au moins un axe de travail obtenu à l'étape iii ;
vi. Détermination de la forme et des dimensions de l'ancillaire à partir des axes de travail, de au moins une surface d'appui et du au moins un moyen de guidage ;
vii. Réalisation de l'ancillaire.

De manière très avantageuse, le procédé selon l'invention permet de réaliser un ancillaire, cet ancillaire étant un ancillaire sur-mesure. De la qualité des images obtenues dépendra le bon positionnement de l'ancillaire et ainsi sa précision. L'acquisition d'images peut être effectuée par tomodensitométrie (TDM) ou par Imagerie par Résonance Magnétique (IRM) ou par la combinaison des CAO des tomodensitométrie/ Imagerie par Résonance Magnétique.

La Tomodensitométrie possède une meilleure résolution spatiale et l'acquisition des images des parties osseuses est plus précise. Dans un mode de réalisation préféré, l'Imagerie par Résonance Magnétique serait utilisée, l'IRM permettant de visualiser le cartilage avec précision. Dans un mode de réalisation encore plus préféré, l'acquisition des images se fait par la combinaison des CAO des tomodensitométrie/Imagerie par Résonance Magnétique.

Lesdites images ainsi acquises seront par la suite segmentées et modélisées en 3D sur ordinateur. La transmission desdites images se fait par encodage. A titre purement illustratif, le format utilisé est le STL (Surface Tesselation Language).

La modélisation en 3D desdites au moins deux surfaces osseuses va permettre de déterminer au moins une surface d'appui sur lesdites surfaces osseuses pour le maintien en position de l'ancillaire, au moins une desdites surface d'appui délimitant au moins une zone de travail dans laquelle on vient modifier localement ladite au moins une portion d'au moins une surface osseuse.

Grâce à la modélisation 3D, il sera par la suite possible de déterminer au moins un axe de travail pour modifier localement au moins une portion d'au moins une desdites surfaces osseuses, et de positionner au moins un moyen de guidage, ledit moyen de guidage étant configuré de manière à guider au moins un dispositif médical le long dudit axe de travail.

Ainsi, et avantageusement, le au moins un moyen de guidage sera dans le prolongement de l'axe de travail déterminé en fonction de l'acte chirurgical, afin de guider le dispositif médical vers la zone de travail, via l'axe de travail.

La modification locale de au moins une des extrémités osseuses peut être choisie parmi l'ostéosynthèse, l'arthrodèse, l'arthroplastie, la cimentoplastie, l'ostéotomie, la neurochirurgie et la kyphoplastie.

On entend par ostéosynthèse, l'ensemble des procédés qui permettent de traiter des fractures ou des problèmes d'ordre mécanique. On citera à titre d'exemple, la pose de broches, de vis, de plaques, de tiges, de clous.

On entend par arthrodèse, l'intervention destinée à fixer une articulation par fusion osseuse et bloquée par ostéosynthèse.

On entend par arthroplastie, l'intervention chirurgicale consistant à rétablir la mobilité d'une articulation en créant un nouvel espace articulaire.

On entend par cimentoplastie, l'intervention chirurgicale consistant à injecter un ciment dans un corps vertébral pathologique.

On entend par kyphoplastie, l'intervention chirurgicale consistant à introduire par voie transcutanée un ballonnet, pouvant être gonflé à plusieurs atmosphères, dans le corps vertébral fracturé.

On entend par ostéotomie, l'acte chirurgical consistant en une coupe diaphysaire d'un os long destiné à réorienter au mieux un axe voir plusieurs axes dudit os afin de repositionner au mieux les articulations sus et sous-jacentes.

Un exemple d'arthroplastie est la réalisation d'un évidement sur au moins une surface osseuse. Dans le cadre de la réalisation d'un évidement, celui-ci sera déterminé par l'homme du métier sur la représentation 3D de au moins une des surfaces osseuses. Dans un mode de réalisation, l'évidement sera être réalisé afin d'être apte et destiné à accueillir un implant ou prothèse. Dans un mode de réalisation encore plus préféré, l'évidemment sera réalisé sur au moins deux surfaces osseuses, de manière à être apte et destiné à accueillir un implant ou une prothèse.

Dans un mode de réalisation, l'implant ou prothèse possède une forme choisie parmi une forme en U, une forme en V, une forme en W et sera un implant personnalisé en fonction de la pathologie et/ou morphologie du patient..

Ainsi, l'extrémité de au moins l'une des surfaces osseuses devra être modifiée localement de manière à être apte et destinée à accueillir un implant ou prothèse, ledit implant ou prothèse ayant une forme choisie parmi une forme en U, en V ou en W. A partir de la forme dudit implant ou prothèse identifiée et ainsi de la projection de la modification locale de l'extrémité de au moins l'une des surfaces osseuses le praticien pourra déterminer au moins un axe de travail de manière à modifier au moins une portion d'au moins une des extrémités osseuses.
- ledit ou au moins un desdits moyens de guidage étant un élément allongé creux, chaque élément allongé creux comportant un canal délimité par une paroi intérieure dudit élément allongé pour le passage d'au moins un dispositif médical au travers dudit élément allongé creux correspondant, à l'étape v), on configure au moins la forme de ladite paroi intérieure de chaque élément allongé creux pour délimiter l' excursion dudit dispositif médical dans la zone de travail correspondante en fonction de la modification locale à apporter dans ladite surface osseuse correspondante.

Le moyen de guidage et la forme du canal intérieur seront ainsi déterminés en fonction de l'action à réaliser dans la zone de travail à la surface de l'extrémité osseuse.

Dans un mode de réalisation, la paroi intérieure de l'extrémité libre, i.e., l'extrémité la plus proche de la surface osseuse, de l'élément allongé creux est évasée ou de forme oblongue, ou de forme tubulaire aplatie, de section droite de forme elliptique, polygonale telle que rectangulaire, carrée.

Ainsi et avantageusement, la surface de déplacement du dispositif médical est configurée de sorte à ce que le déplacement de l'outil sur cette surface détermine le pourtour de la modification locale à apporter sur la surface osseuse.
- Le procédé de fabrication de l'ancillaire comprend en outre une étape supplémentaire de fabrication additive des au moins deux surfaces osseuses acquises à l'étape ii.

Avantageusement, la fabrication additive des au moins deux surfaces osseuse permet de vérifier l'adéquation de l'ancillaire de guidage au sens de la présente invention, avec les au moins deux surfaces osseuses.
- L'ancillaire est réalisé par fabrication additive.

La fabrication additive est choisie parmi les techniques de stéréolithographie, frittage laser de poudre (Selective Laser Sintering, SLS), dépôt de fils fondus (Fused Deposition Modeling, FDM), le frittage Laser de métal (Selective Laser Melting, SLM), ou tout procédé de fabrication additive tel que l'EBM (Electron Beam Melting). L'invention concerne également un ancillaire au sens de la présente invention pour son utilisation dans l'ostéosynthèse, l'arthrodèse, l'arthroplastie, la cimentoplastie, une ostéotomie, une biopsie, la neurochirurgie et la kyphoplastie.

A titre d'exemple, la présente demande concerne également une méthode de traitement d'un sujet en ayant besoin, ladite méthode comprenant l'utilisation d'un ancillaire pour la réalisation d'une ostéosynthèse, arthrodèse, arthroplastie, cimentoplastie, ostéotomie, biopsie et /ou kyphoplastie.
- L'ancillaire est utilisé pour la réalisation d'un évidement sur au moins une surface osseuse.
- A titre d' exemple les deux surfaces osseuses correspondent à la zone de juxtaposition définissant une articulation.

A titre purement illustratif, on citera, les articulations intervertébrales, l'articulation lombo sacrée, l'articulation sacro-coccygienne, les articulations inter coccygiennes, les articulations sacro-iliaques, la symphyse pubienne, l'articulation gléno-humérale, l'articulation acromio-claviculaire, l'articulation huméro-ulnaire, l'articulation huméro-radiale, l'articulation radio-ulnaire proximale, l'articulation radio-carpienne, l'articulation radio-ulnaire distale, les articulations entre les os du carpe, les articulations carpo-métacarpiennes, les articulations inter métacarpiennes, les articulations métacarpo-phalangiennes, les articulations inter phalangiennes, l'articulation coxo-fémorale, l'articulation tibio-fémorale, l'articulation patellofémorale, l'articulation tibiofibulaire proximale, l'articulation talo-crurale, l'articulation tibiofibulaire distale, les articulations entres les os du tarse, les articulations tarsométatarsiennes, les articulations inter-métatarsiennes, les articulations métatarso-phalangiennes, les articulations interphalangiennes.

A titre d'exemple, l'ancillaire est destiné à coopérer avec l'articulation tibio-fémorale.

A titre d'exemple, l'ancillaire est destiné à coopérer avec l'articulation coxo-fémorale.

Dans un autre exemple, les articulations concernées sont celles du rachis lombaire et cervical.

Dans un autre exemple, l'articulation est l'articulation de l'épaule.

Enfin, dans un autre exemple, l'ancillaire est utilisé pour coopérer avec au moins l'une des vertèbres du rachis cervical.

Dans un exemple, l'ancillaire de guidage est utilisé dans la réalisation d'un évidement, préférentiellement pour la réalisation d'un évidement apte à recevoir un implant, ledit implant étant une prothèse totale de genou (PTG), plus préférentiellement une prothèse totale de genou de resurfaçage personnalisée.

La présente demande concerne également un ensemble comprenant un ancillaire de guidage au sens de la présente invention et au moins un dispositif médical

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels :
La Figure 1 est une vue en perspective de l'extrémité proximale du tibia et de l'extrémité distale du fémur, ainsi que de l'ancillaire selon un premier mode de réalisation, ledit ancillaire étant d'un seul tenant et coopérant avec ladite extrémité proximale du tibia et ladite extrémité distale du fémur.
La Figure 2 est une photographie prise en vue de face et montrant la surface d'appui tibiale
La Figure 3 est une photographie prise en vue de dessous de l'ancillaire selon un premier mode de réalisation, montrant la surface d'appui fémorale

### DESCRIPTION DETAILLEE DE L'INVENTION

Tout d'abord, on note que les figures ne sont pas à l'échelle.

### PREMIER MODE DE REALISATION : ANCILLAIRE POUR POSE D'UN IMPLANT DE RESURFACAGE DU GENOU

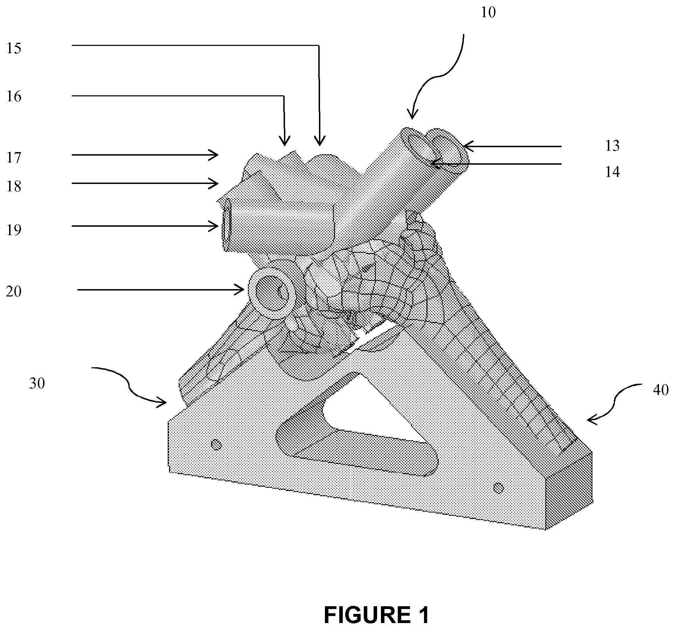

La figure 1 est une vue en perspective d'un ancillaire 10 de guidage selon un premier mode de réalisation destiné à coopérer avec au moins deux surfaces osseuses, lesdites deux surfaces osseuses correspondant à l'extrémité proximale 30 du tibia et l'extrémité distale 40 du fémur.

L'ancillaire 10 de guidage comprend huit moyens de guidage, lesdits moyens de guidage étant des éléments tubulaires 13, 14, 15, 16, 17, 18, 19, 20. Les éléments tubulaires 13, 14, 15, 16, 17, 18, 19, 20 de l'ancillaire 10 de guidage sont aptes à recevoir au moins un dispositif médical.

De manière avantageuse, l'ancillaire de guidage d'un seul tenant selon ce premier mode de réalisation épouse parfaitement l'extrémité proximale du tibia et l'extrémité distale du fémur et permet de maintenir l'ancillaire 10 de guidage en position. De manière encore plus avantageuse, son positionnement en préopératoire ne nécessite pas la luxation de l'articulation tibio-fémorale, ni de sacrifice ligamentaire.

L'ancillaire 10 de guidage selon ce premier mode de réalisation, ainsi que les huit éléments tubulaires 13, 14, 15, 16, 17, 18, 19, 20 de l'ancillaire 10 de guidage sont réalisés en polyamide 12 biocompatible ou en titane selon la nécessité de résistance de l'ancillaire.

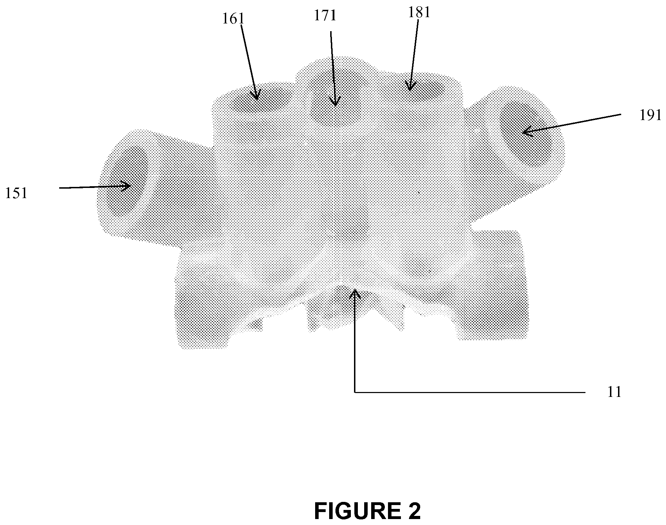

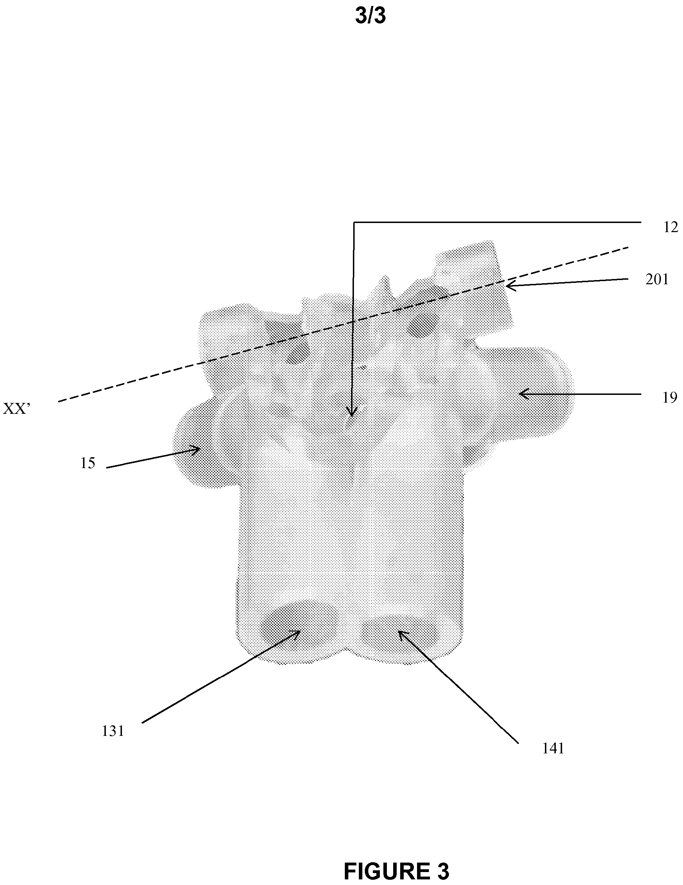

La Figure 2 est une photographie prise en vue de face et montrant la surface d'appui tibiale. La Figure 3 est une photographie prise en vue de dessous de l'ancillaire selon un premier mode de réalisation, montrant la surface d'appui fémorale

Les éléments représentés sur la Figure 2 et portant les mêmes références que ceux de la Figure 1 représentent les mêmes objets, lesquels ne sont pas décrits de nouveau ci-dessous.

L'ancillaire 10 de guidage comprend pour l'extrémité proximale du tibia une surface d'appui tibiale 11 destinée à épouser une partie de l'extrémité proximale du tibia et pour l'extrémité distale du fémur, une surface d'appui fémorale 12 destinée à épouser l'extrémité distale fémorale, lesdites surfaces d'appui tibiale 11 et fémorale 12 permettent avantageusement de maintenir en position l'ancillaire 10 sur respectivement l'extrémité proximale du tibia et l'extrémité distale du fémur. La surface d'appui tibiale 11 et la surface d'appui fémorale 12 délimitent chacune une zone de travail à la surface de chacune des extrémités osseuses, respectivement l'extrémité distale du fémur pour la surface d'appui fémorale 12 et l'extrémité proximale du tibia pour la surface d'appui tibiale 11.

Ainsi, selon ce premier mode de réalisation, l'ancillaire 10 de guidage comprend huit éléments tubulaires 13, 14, 15, 16, 17, 18, 19, 20, chacun des éléments tubulaires présente une lumière 131, 141, 151, 161, 171, 181, 191, 201, la lumière de chacun des éléments tubulaires 13, 14, 15, 16, 17, 18, 19, 20 permet le déplacement rectiligne d'au moins un dispositif médical selon un axe longitudinal.

Selon ce premier mode de réalisation, l'ancillaire 10 de guidage permet de procéder à des évidements à l'extrémité de la surface osseuse proximale tibiale et à l'extrémité de la surface osseuse distale fémorale.

Selon ce mode de réalisation, les évidements à l'extrémité de la surface osseuse proximale tibiale et à l'extrémité de la surface osseuse distale fémorale sont aptes et destinés à accueillir une prothèse totale de genou (PTG).

Ainsi, l'ancillaire 10 de guidage selon le premier mode de réalisation comprenant huit éléments tubulaires 13, 14, 15, 16, 17, 18, 19, 20, chacun des éléments tubulaires correspond à un axe de travail pour guider un dispositif médical sur chacune des deux zones de travail, respectivement la surface d'appui 11 tibiale et la surface d'appui 12 fémorale afin de modifier localement au moins une portion de chacune des extrémités osseuses dans chacune desdites zone de travail afin de pratiquer un évidement.

Chacun des axes de travail est confondu avec l'axe principal de la lumière 131, 141, 151, 161, 171, 181, 191, 201de chacun des éléments tubulaires 13, 14, 15, 16, 17, 18, 19, 20 de l'ancillaire 10 de guidage, l'axe de travail de chacun des éléments tubulaires 13, 14, 15, 16, 17, 18, 19, 20, étant dirigé selon un axe tangent ou sensiblement tangent en un point de la surface osseuse proximale fémorale et de la surface osseuse distale tibiale.

Un axe de travail XX' est représenté sur la figure 3.

Selon ce premier mode de réalisation, l'ancillaire de guidage est utilisé pour la réalisation d'un évidement, ledit évidement étant apte et destiné à accueillir une prothèse totale de genou. Ainsi, l'ancillaire selon ce premier mode de réalisation est utilisé pour guider au moins un dispositif médical permettant de réaliser au moins un évidement à l'extrémité distale tibiale et au moins un évidement à l'extrémité proximale fémorale.

L'imagerie est réalisée par Imagerie par Résonnance Magnétique de l'articulation tibio-fémorale, et plus précisément de l'extrémité proximale tibiale et de l'extrémité distale fémorale.

Les images des surfaces osseuses ainsi acquises par Imagerie par Résonnance Magnétique seront par la suite segmentées et modélisées en 3D sur un ordinateur, la transmission desdites images étant réalisée sous format STL. Une imagerie 3D de l'articulation tibio-fémorale, i.e. de l'extrémité proximale tibiale et de l'extrémité distale fémorale est ainsi acquise.

Sur la base de cette imagerie 3D de l'articulation tibio-fémorale, le praticien va déterminer les évidements nécessaires pour accueillir une prothèse totale de genou, et donc pour modifier localement les extrémités osseuses proximale fémorale et distale tibiale.

On entend par praticien au sens de la présente invention un ingénieur / prothésiste.

Selon ce mode de réalisation, la prothèse totale de genou correspond à un implant fémoral sous forme de U ainsi qu'à un implant tibial sous forme de U.

L'évidement envisagé est l'empreinte de chacun des implants, de sorte que la surface externe de chacun des implants soit affleurante ou sensiblement affleurante à la surface osseuse des extrémités tibiale et fémorale. L'implant dans ce mode de réalisation est un implant sur-mesure adaptée à la morphologie / pathologie du patient.

A partir dudit évidement projeté sur l'imagerie 3D de l'articulation tibio-fémorale, huit axes de travail vont être déterminés pour la réalisation de l'évidement à la surface respectivement des extrémités osseuses distale fémorale et proximale tibiale. Deux axes vont être dirigés vers les condyles fémoraux, respectivement le condyle médial et le condyle latéral. Un axe va être dirigé vers la surface patellaire de l'extrémité distale fémorale. Deux axes vont avoir pour direction et respectivement, la zone de juxtaposition entre le condyle médial de l'extrémité distale du fémur et le plateau tibial de l'extrémité proximale du fémur et la zone de juxtaposition entre le condyle latéral de l'extrémité distale du fémur et le plateau latéral de l'extrémité proximale du tibia. Deux axes auront pour direction respectivement le plateau tibial médial et le plateau tibial latéral de l'extrémité proximale du tibia. Un axe passera par la zone de juxtaposition du plateau tibial médial et du condyle fémorale médiale ainsi que par la zone de juxtaposition entre le plateau tibial latéral et le condyle latéral fémoral. Cet axe est représenté sur la Figure 3, comme étant l'axe XX'.

A partir des axes de travail modifiant la surface de l'extrémité proximale fémorale, la surface de travail de l'extrémité proximale fémorale va être déterminée et à partir des axes de travail modifiant l'extrémité distale tibiale, la surface de travail de l'extrémité distale tibiale va être déterminée.

La surface de travail de l'extrémité distale tibiale et la surface de travail de l'extrémité proximale fémorale correspondent ainsi respectivement à la surface d'appui tibiale 11 et à la surface d'appui fémorale 12 de l'ancillaire 10 de guidage.

Les huit éléments tubulaires 13, 14, 15, 16, 17, 18, 19, 20 vont être positionnés de sorte à ce que l'axe longitudinal de la lumière 131, 141, 151, 161, 171, 181, 191, 201 de chacun des éléments tubulaires coïncident avec les huit axes de travail déterminés par le praticien.

La Figure 1 est une représentation de l'articulation tibio fémorale droite d'un patient, lorsque l'articulation est à 90°.

Aussi, l'axe longitudinal de la lumière 131 de l'élément tubulaire 13 correspondra sensiblement à l'axe de de travail passant par le condyle latéral de l'extrémité distale fémorale.

L'axe longitudinal de la lumière 141 de l'élément tubulaire 14 de l'ancillaire correspondra sensiblement à l'axe de travail passant par le condyle médial de l'extrémité distale fémorale.

L'axe longitudinal de la lumière 171 de l'élément tubulaire 17 correspondra sensiblement à l'axe de travail dirigé vers la surface patellaire de l'extrémité distale fémorale.

L'axe longitudinal de la lumière 201 de l'élément tubulaire 20 correspondra à l'axe de travail XX' dirigé et traversant respectivement la zone de juxtaposition du plateau tibial médial et du condyle fémorale médiale ainsi que par la zone de juxtaposition entre le plateau tibial latéral et le condyle latéral fémoral et ce afin de réaliser un évidemment affleurant la surface glénoïde du plateau tibial et la surface du condyle fémoral.

L'axe longitudinal de la lumière 151 de l'élément tubulaire 15 correspondra sensiblement à l'axe de travail dirigé vers la zone de juxtaposition entre le condyle latéral de l'extrémité distale du fémur et le plateau latéral de l'extrémité proximale du tibia et ce afin de réaliser un évidemment affleurant la surface glénoïde du plateau tibial et la surface du condyle fémoral.

L'axe longitudinal a lumière 191 de l'élément tubulaire 19 correspondra sensiblement à l'axe de travail de la zone de juxtaposition entre le condyle médial de l'extrémité distale du fémur et le plateau tibial de l'extrémité proximale du fémur. L'axe longitudinal de la lumière 161 de l'élément tubulaire 16 correspondra sensiblement à l'axe de travail dirigé vers le plateau tibial latéral de l'extrémité proximale du tibia.

Enfin, l'axe longitudinal de la lumière 181 de l'élément tubulaire 18 correspondra sensiblement à l'axe de travail dirigé vers le plateau tibial médial de l'extrémité proximale du tibia.

Le dispositif médical selon le premier mode de réalisation est un dispositif de fraisage.

Les huit éléments tubulaires 13, 14, 15, 16, 17, 18, 19, 20, comprenant chacun une lumière 131, 141, 151, 161, 171, 181, 191, 201 comportant un axe longitudinal correspondant à chacun des huit axes de travail préalablement déterminés à la surface de la surface osseuse, ainsi que la surface d'appui tibiale 11 et la surface d'appui fémorale 12 de l'ancillaire 10 de guidage, correspondant aux surfaces de travail de l'extrémité distale tibiale et de l'extrémité proximale fémorale vont ainsi être déterminées afin de réaliser l'ancillaire 10 de guidage par impression 3D, selon la technique soit de stéréolithographie si l'ancillaire doit être réalisé en titane ou en inox 316L, soit par frittage de poudre si l'ancillaire doit être réalisé en polyamide 12.

Ainsi, l'ancillaire selon ce premier mode de réalisation est utilisé dans la réalisation d'un évidement pour la pose d'une prothèse totale du genou.

L'ancillaire 10 de guidage est donc destiné à coopérer avec l'extrémité proximale 40 fémorale et l'extrémité distale 30 tibiale, et comprend :
- pour l'extrémité distale 40 du fémur, une surface d'appui fémorale 12 destinée à épouser l'extrémité distale 40 fémorale, pour l'extrémité proximale 30 du tibia une surface d'appui tibiale 11 destinée à épouser une partie de l'extrémité proximale 30 du tibia, lesdites surfaces d'appui 11 et 12 délimitant chacune une zone de travail à l'extrémité distale du fémur pour la surface d'appui fémorale 12 et l'extrémité proximale du tibia pour la surface d'appui tibiale 11 ;
- huit éléments tubulaires 13, 14, 15, 16, 17, 18, 19, 20 aptes à recevoir un dispositif de fraisage, lesdits huit éléments tubulaires guidant le dispositif de fraisage vers les deux zones de travail, l'une à l'extrémité distale du fémur délimitée par la surface d'appui fémorale 12 et l'une à l' extrémité proximale du tibia pour la surface d'appui tibiale 11.

## Revendications

1. Ancillaire de guidage (10) destiné à coopérer avec au moins deux surfaces osseuses (11, 12) **caractérisé en ce qu'**il comprend :
- Pour chaque surface osseuse, une surface d'appui qui est une empreinte d'une portion de la surface osseuse correspondante pour venir épouser au moins en partie une portion de la surface osseuse correspondante ; ladite surface d'appui délimitant une zone de travail sur ladite surface osseuse lorsqu'elle est en contact avec ladite surface osseuse ;
- lesdites surfaces d'appui étant configurées pour maintenir en position ledit ancillaire sur les surfaces osseuses correspondantes et
- Au moins un moyen de guidage (13, 14, 15, 16, 17, 18, 19, 20) apte à recevoir au moins un dispositif médical, ledit au moins un moyen de guidage guidant le dispositif médical vers ladite zone de travail sur la surface osseuse correspondante, chaque moyen de guidage définissant un axe de travail (131, 141, 151, 161,171, 181, 191) pour le déplacement du dispositif médical le long dudit axe de travail, ladite zone de travail étant déterminée par lesdits axes de travail.

2. Ancillaire de guidage selon la revendication 1, **caractérisé en ce que** l'ancillaire comporte au moins deux parties d'ancillaire, chacune des parties d'ancillaire étant destinée à coopérer avec au moins une surface osseuse distincte.

3. Ancillaire de guidage selon la revendication 2, **caractérisé en ce que** l'ancillaire comprend au moins un moyen d'assemblage permettant de lier lesdites au moins deux parties d'ancillaire.

4. Ancillaire de guidage selon la revendication 3, **caractérisé en ce que** chaque partie d'ancillaire comporte une portion d'assemblage femelle et/ou une portion d'assemblage mâle, deux parties d'ancillaire destinées à être immédiatement contiguës lorsque ledit ancillaire est assemblé, présentant une zone d'assemblage femelle et une zone d'assemblage mâle destinées à coopérer entre elles pour assurer l'assemblage desdites deux parties d'ancillaire, au moins certaines desdites parties d'ancillaire comprenant au moins un moyen de guidage, lesdits moyens de guidage étant distincts **ou** au moins certaines desdites parties d'ancillaire comprenant une portion de moyen de guidage, lesdites portions de moyen de guidage étant distinctes et destinées à former un moyen de guidage lorsque lesdites parties d'ancillaires sont assemblées entre elles.

5. Ancillaire de guidage selon la revendication 4, **caractérisé en ce que** lesdites parties d'ancillaire comprennent, pour chaque zone d'assemblage mâle ou femelle, au moins un moyen détrompeur pour empêcher l'assemblage de parties d'ancillaire non destinées à être immédiatement adjacentes lorsque l'ancillaire est assemblé.

6. Ancillaire de guidage selon l'une quelconque des revendications 2 à 5, **caractérisé en ce que** lesdites parties d'ancillaire sont configurées pour définir au moins 2 axes de guidage d'un ou plusieurs dispositifs médicaux différents lorsqu'elles sont assemblées entre elles.

7. Ancillaire de guidage selon les revendications 1 à 6, **caractérisé en ce que** chaque partie dudit ancillaire comprend pour chaque surface osseuse une surface d'appui qui est une empreinte d'une portion de la surface osseuse correspondante pour venir épouser au moins en partie une portion de ladite surface osseuse correspondante, ladite surface d'appui délimitant une zone de travail sur ladite surface osseuse, chaque partie comprenant au moins deux moyens de guidage, lesdits au moins deux moyens de guidage définissant des axes de travail distincts, pour guider le déplacement d'au moins un dispositif médical le long desdits axes de travail sur la surface osseuse correspondante.

8. Ancillaire de guidage selon l'une quelconque des revendications 1 à 7, **caractérisé en ce que** chaque surface d'appui est une empreinte de la portion de la surface osseuse qu'elle est destinée à épouser, ladite surface osseuse étant choisie parmi la surfaces osseuse de l'extrémité distale fémorale et de l'extrémité proximale tibiale, la surfaces osseuse de l'extrémité distale fémorale et de l'extrémité proximale tibiale, la surface osseuse des vertèbres lombaires et cervicales, la surface osseuse de l'acromion de l'omoplate et la surface osseuse de la tête de l'humérus, la surface osseuse du fémur.

9. Ancillaire de guidage selon l'une quelconque des revendications précédentes, **caractérisé en ce que** ledit ou au moins un desdits moyens de guidage est configuré pour définir une butée pour le déplacement dudit dispositif de guidage correspondant.

10. Ancillaire selon l'une quelconque des revendications précédentes **caractérisé en ce que** le au moins un moyen de guidage est choisi parmi un élément allongé creux, un élément tubulaire, des fentes orientées, un chemin de déplacement, et des combinaisons de ces éléments.

11. Ancillaire selon l'une quelconque des revendications précédentes, **caractérisé en ce que** chaque moyen de guidage est un élément tubulaire, l'élément tubulaire comporte une lumière définissant un axe longitudinal pour le déplacement d'au moins un dispositif médical le long dudit axe.

12. Ancillaire selon l'une quelconque des revendications précédentes, **caractérisé en ce que** chaque élément allongé creux comporte un canal pour le passage d'au moins un dispositif médical au travers dudit élément allongé creux correspondant, la paroi intérieure dudit élément allongé creux délimitant ledit canal définissant une surface de déplacement dudit dispositif médical contre au moins une partie de laquelle est destinée à être déplacé ledit dispositif médical afin de délimiter l'excursion dudit dispositif médical dans ladite zone de travail de ladite surface osseuse correspondante.

13. Procédé de fabrication d'un ancillaire de guidage selon l'une quelconque des revendications précédentes, **caractérisé en ce qu'**il comprend les étapes suivantes :
i.Acquisition d'une ou plusieurs images par tomodensitométrie et/ou Imagerie par Résonnance Magnétique, de au moins deux surfaces osseuses ;
ii. Représentation en 3D de l'imagerie des au moins deux surfaces osseuses acquises à l'étape i. ;
iii. Détermination d'au moins un axe de travail pour modifier localement au moins une portion d'au moins une desdites surfaces osseuses;
iv. Détermination d'au moins une surface d'appui sur lesdites surfaces osseuses pour le maintien en position de l'ancillaire, au moins une desdites surfaces d'appui délimitant au moins une zone de travail dans laquelle on cherche à modifier localement ladite au moins une portion d'au moins une surface osseuse ;
v. Positionnement d'au moins un moyen de guidage, ledit au moins un moyen de guidage étant configuré de manière à guider au moins un dispositif médical le long dudit au moins un axe de travail obtenu à l'étape iii ;
vi. Détermination de la forme et des dimensions de l'ancillaire à partir des axes de travail, de au moins une surface d'appui et du au moins un moyen de guidage ;
vii. Réalisation de l'ancillaire.

14. Procédé selon la revendication 13, **caractérisé en ce que** ledit ou au moins un desdits moyens de guidage étant un élément allongé creux, chaque élément allongé creux comportant un canal délimité par une paroi intérieure dudit élément allongé pour le passage d'au moins un dispositif médical au travers dudit élément allongé creux correspondant, à l'étape v), on configure au moins la forme de ladite paroi intérieure de chaque élément allongé creux pour délimiter l'excursion dudit dispositif médical dans la zone de travail correspondante en fonction de la modification locale à apporter dans ladite surface osseuse correspondante.

15. Procédé de fabrication d'un ancillaire de guidage selon les revendications 13 ou 14, **caractérisé en ce que** l'ancillaire est réalisé par fabrication additive.

## Patentansprüche

1. Führungshilfsvorrichtung (10), welche dazu bestimm ist, mit wenigstens zwei Knochenflächen (11, 12) zusammenzuwirken, **dadurch gekennzeichnet, dass** diese umfasst:
- für jede Knochenfläche eine Auflagefläche, die ein Abdruck eines Abschnitts der entsprechenden Knochenfläche ist, um wenigstens teilweise einen Abschnitt der entsprechenden Knochenfläche zu umschließen; wobei die Auflagefläche einen Arbeitsbereich auf der Knochenfläche begrenzt, wenn sie mit der Knochenfläche in Kontakt steht;
- wobei die Auflageflächen dazu ausgebildet sind, die Hilfsvorrichtung auf den entsprechenden Knochenflächen in Position zu halten, und
- wenigstens ein Führungsmittel (13, 14, 15, 16, 17, 18, 19, 20), das wenigstens eine medizinische Vorrichtung aufnehmen kann, wobei das wenigstens eine Führungsmittel die medizinische Vorrichtung zu dem Arbeitsbereich auf der entsprechenden Knochenfläche führt, wobei jedes Führungsmittel eine Arbeitsachse (131, 141, 151, 161, 171, 181, 191) für die Bewegung der medizinischen Vorrichtung entlang der Arbeitsachse definiert, wobei der Arbeitsbereich durch die Arbeitsachsen bestimmt wird.

2. Führungshilfsvorrichtung nach Anspruch 1, **dadurch gekennzeichnet, dass** die Hilfsvorrichtung wenigstens zwei Hilfsvorrichtungsteile umfasst, wobei jeder Hilfsvorrichtungsteil dazu bestimmt ist, mit wenigstens einer unterschiedlichen Knochenfläche zusammenzuwirken.

3. Führungshilfsvorrichtung nach Anspruch 2, **dadurch gekennzeichnet, dass** die Hilfsvorrichtung wenigstens ein Verbindungsmittel umfasst, das es ermöglicht, die wenigstens zwei Hilfsvorrichtungsteile miteinander zu verbinden.

4. Führungshilfsvorrichtung nach Anspruch 3, **dadurch gekennzeichnet, dass** jeder Hilfsvorrichtungsteil einen weiblichen Verbindungsabschnitt und/oder einen männlichen Verbindungsabschnitt umfasst, wobei zwei Hilfsvorrichtungsteile, die dazu bestimmt sind, unmittelbar aneinander anzugrenzen, wenn die Hilfsvorrichtung zusammengebaut ist, einen weiblichen Verbindungsbereich und einen männlichen Verbindungsbereich aufweisen, die dazu bestimmt sind zusammenzuwirken, um die Verbindung der beiden Hilfsvorrichtungsteile zu gewährleisten, wobei wenigstens einige Hilfsvorrichtungsteile wenigstens ein Führungsmittel umfassen, wobei die Führungsmittel voneinander getrennt sind, **oder** wobei wenigstens einige der Hilfsvorrichtungsteile einen Teil eines Führungsmittels umfassen, wobei die Führungsmittelteile voneinander getrennt sind und dazu bestimmt sind, ein Führungsmittel zu bilden, wenn die Hilfsvorrichtungsteile miteinander verbunden sind.

5. Führungshilfsvorrichtung nach Anspruch 4, **dadurch gekennzeichnet, dass** die Hilfsvorrichtungsteile für jeden männlichen oder weiblichen Verbindungsbereich wenigstens ein Unverwechselbarkeitsmittel umfassen, um zu verhindern, dass Hilfsvorrichtungsteile miteinander verbunden werden, die nicht dazu bestimmt sind, unmittelbar nebeneinander zu liegen, wenn die Hilfsvorrichtung zusammengebaut ist.

6. Führungshilfsvorrichtung nach einem der Ansprüche 2 bis 5, **dadurch gekennzeichnet, dass** die Hilfsvorrichtungsteile dazu ausgebildet sind, bei der Verbindung miteinander wenigstens zwei Führungsachsen für ein oder mehrere verschiedene medizinische Vorrichtungen zu definieren.

7. Führungshilfsvorrichtung nach den Ansprüchen 1 bis 6, **dadurch gekennzeichnet, dass** jedes Teil der Hilfsvorrichtung für jede Knochenfläche eine Auflagefläche umfasst, die ein Abdruck eines Abschnitts der entsprechenden Knochenfläche ist, um wenigstens teilweise einen Abschnitt der entsprechenden Knochenfläche zu umschließen, wobei die Auflagefläche einen Arbeitsbereich auf der Knochenfläche begrenzt, wobei jeder Teil wenigstens zwei Führungsmittel umfasst, wobei die wenigstens zwei Führungsmittel unterschiedliche Arbeitsachsen definieren, um die Bewegung wenigstens einer medizinischen Vorrichtung entlang der Arbeitsachsen auf der entsprechenden Knochenfläche zu führen.

8. Führungshilfsvorrichtung nach einem der Ansprüche 1 bis 7, **dadurch gekennzeichnet, dass** jede Auflagefläche ein Abdruck des Teils der Knochenfläche ist, den sie umschließen soll, wobei die Knochenfläche gewählt ist aus der Knochenfläche des distalen Endes des Femurs und des proximalen Endes der Tibia, der Knochenfläche des distalen Endes des Femurs und des proximalen Endes der Tibia, der Knochenfläche der Lenden- und Halswirbel, der Knochenfläche des Schulterblatts und der Knochenfläche des Humeruskopfes, der Knochenfläche des Femurs.

9. Führungshilfsvorrichtung nach einem der vorstehenden Ansprüche, **dadurch gekennzeichnet, dass** die wenigstens eine Führungsvorrichtung dazu ausgebildet ist, einen Anschlag für die Bewegung der entsprechenden Führungsvorrichtung zu definieren.

10. Hilfsvorrichtung nach einem der vorstehenden Ansprüche, **dadurch gekennzeichnet, dass** die wenigstens eine Führungsvorrichtung aus einem hohlen länglichen Element, einem rohrförmigen Element, ausgerichteten Schlitzen, einer Bewegungsbahn und Kombinationen dieser Elemente gewählt ist.

11. Hilfsvorrichtung nach einem der vorstehenden Ansprüche, **dadurch gekennzeichnet, dass** jedes Führungsmittel ein rohrförmiges Element ist, wobei das rohrförmige Element eine Öffnung aufweist, die eine Längsachse für die Bewegung wenigstens einer medizinischen Vorrichtung entlang dieser Achse definiert.

12. Hilfsvorrichtung nach einem der vorstehenden Ansprüche, **dadurch gekennzeichnet, dass** jedes längliche hohle Element einen Kanal für den Durchgang wenigstens einer medizinischen Vorrichtung durch das entsprechende längliche hohle Element aufweist, wobei die Innenwand des hohlen länglichen Elements den Kanal begrenzt, der eine Bewegungsfläche der medizinischen Vorrichtung definiert, gegen deren wenigstens einen Teil die medizinischen Vorrichtung bewegt werden soll, um die Auslenkung der medizinischen Vorrichtung in dem Arbeitsbereich der entsprechenden Knochenfläche zu begrenzen.

13. Verfahren zur Herstellung einer Führungsvorrichtung nach einem der vorstehenden Ansprüche, **dadurch gekennzeichnet, dass** es die folgenden Schritte umfasst:
i. Erfassung eines oder mehrerer Bilder mittels Computertomographie und/oder Magnetresonanztomographie von wenigstens zwei Knochenflächen;
ii. 3D-Darstellung der in Schritt i) erworbenen Bilder der wenigstens zwei Knochenflächen;
iii. Bestimmung wenigstens einer Arbeitsachse, um wenigstens einen Abschnitt wenigstens einer der Knochenflächen lokal zu verändern;
iv. Bestimmung wenigstens einer Auflagefläche auf den genannten Knochenflächen zum Halten de r Hilfsvorrichtung in Position, wobei wenigstens eine der genannten Auflageflächen wenigstens einen Arbeitsbereich begrenzt, in dem der wenigstens eine Abschnitt der wenigstens einen Knochenfläche lokal verändert werden soll;
v. Positionierung wenigstens eines Führungsmittels, wobei das wenigstens eine Führungsmittel dazu ausgebildet ist, wenigstens eine medizinische Vorrichtung entlang der wenigstens einen in Schritt iii erhaltenen Arbeitsachse zu führen;
vi. Bestimmung der Form und der Abmessungen der Hilfsvorrichtungsteil anhand der Arbeitsachsen, wenigstens einer Auflagefläche und wenigstens eines Führungsmittels;
vii. Herstellung der Hilfsvorrichtung.

14. Verfahren nach Anspruch 13, **dadurch gekennzeichnet, dass** die wenigstens eine Führungsvorrichtung ein längliches hohles Element ist, wobei jedes längliche hohle Element einen von einer Innenwand des länglichen Elements begrenzten Kanal für den Durchgang wenigstens einer medizinischen Vorrichtung durch das entsprechende längliche hohle Element aufweist, wobei in Schritt v) wenigstens die Form der Innenwand jedes länglichen hohlen Elements so gestaltet ist, dass sie die Auslenkung der medizinischen Vorrichtung im entsprechenden Arbeitsbereich entsprechend der vorzunehmenden lokalen Veränderung der entsprechenden Knochenfläche begrenzt.

15. Verfahren zur Herstellung eines Führungshilfsmittels nach Anspruch 13 oder 14, **dadurch gekennzeichnet, dass** das Hilfsmittel durch additive Fertigung hergestellt wird.

## Claims

1. A guiding ancillary (10) intended to cooperate with at least two bone surfaces (11, 12), **characterised in that** it comprises:
- For each bone surface, a bearing surface which is an impression of a portion of the corresponding bone surface to at least partially match a portion of the corresponding bone surface; said bearing surface delimiting a working area on said bone surface when it is in contact with said bone surface;
- said bearing surfaces being configured to hold said ancillary in position on the corresponding bone surfaces and
- At least one guide means (13, 14, 15, 16, 17, 18, 19, 20) capable of receiving at least one medical device, said at least one guide means guiding the medical device towards said working area on the corresponding bone surface, each guide means defining a working axis (131, 141, 151, 161, 171, 181, 191) for moving the medical device along said working axis, said working area being determined by said working axes.

2. The guiding ancillary according to claim 1, **characterised in that** the ancillary includes at least two ancillary parts, each of the ancillary parts being intended to cooperate with at least one distinct bone surface.

3. The guiding ancillary according to claim 2, **characterised in that** the ancillary comprises at least one assembly means allowing connecting said at least two ancillary parts.

4. The guiding ancillary according to claim 3, **characterised in that** each ancillary part includes a female assembly portion and/or a male assembly portion, two ancillary parts intended to be immediately contiguous when said ancillary is assembled, having a female assembly area and a male assembly area intended to cooperate together to ensure the assembly of said two ancillary parts, at least some of said ancillary parts comprising at least one guide means, said guide means being distinct or at least some of said ancillary parts comprising a guide means portion, said guide means portions being distinct and intended to form a guide means when said ancillary parts are assembled together.

5. The guiding ancillary according to claim 4, **characterised in that** said ancillary parts comprise, for each male or female assembly area, at least one foolproof means to prevent assembly of ancillary parts not intended to be immediately adjacent when the ancillary is assembled.

6. The guiding ancillary according to any one of claims 2 to 5, **characterised in that** said ancillary parts are configured to define at least 2 guide axes of one or more different medical device(s) when they are assembled together.

7. The guiding ancillary according to claims 1 to 6, **characterised in that** each part of said ancillary comprises for each bone surface a bearing surface which is an impression of a portion of the corresponding bone surface to at least partially match a portion of said corresponding bone surface, said bearing surface delimiting a working area on said bone surface, each part comprising at least two guide means, said at least two guide means defining distinct working axes, for guiding the movement of at least one medical device along said working axes on the corresponding bone surface.

8. The guiding ancillary according to any one of claims 1 to 7, **characterised in that** each bearing surface is an impression of the portion of the bone surface it is intended to match, said bone surface being selected from among the bone surfaces of the femoral distal end and the tibial proximal end, the bone surfaces of the femoral distal end and of the tibial proximal end, the bone surface of the lumbar and cervical vertebrae, the bone surface of the acromion of the scapula and the bone surface of the humeral head, the bone surface of the femur.

9. The guiding ancillary according to any one of the preceding claims, **characterised in that** said or at least one of said guide means is configured to define a stop for the movement of said corresponding guide device.

10. The ancillary according to any one of the preceding claims, **characterised in that** the at least one guide means is selected from among a hollow elongate element, a tubular element, oriented slots, a movement track, and combinations of these elements.

11. The ancillary according to any one of the preceding claims, **characterised in that** each guide means is a tubular element, the tubular element includes an aperture defining a longitudinal axis for the movement of at least one medical device along said axis.

12. The ancillary according to any one of the preceding claims, **characterised in that** each hollow elongate element includes a channel for the passage of at least one medical device throughout said corresponding hollow elongate element, the internal wall of said hollow elongate element delimiting said channel defining a surface for moving said medical device against at least part of which said medical device is intended to be moved in order to delimit the excursion of said medical device in said working area of said corresponding bone surface.

13. A method for manufacturing a guiding ancillary according to any one of the preceding claims, **characterised in that** it comprises the following steps:
i. Acquisition of one or more image(s) by computed tomography and/or Magnetic Resonance Imaging of at least two bone surfaces;
ii. 3D representation of the imaging of the at least two bone surfaces acquired in step i.;
iii. Determination of at least one working axis to locally modify at least a portion of at least one of said bone surfaces;
iv. Determination of at least one bearing surface on said bone surfaces for holding the ancillary in position, at least one of said bearing surfaces delimiting at least one working area in which it is sought to locally modify said at least a portion of at least one bone surface;
v. Positioning of at least one guide means, said at least one guide means being configured so as to guide at least one medical device along said at least one working axis obtained in step iii;
vi. Determination of the shape and of the dimensions of the ancillary from the working axes, of at least one bearing surface and of the at least one guide means;
vii. Making of the auxiliary.

14. The method according to claim 13, **characterised in that** said or at least one of said guide means is a hollow elongate element, each hollow elongate element including a channel delimited by an internal wall of said elongate element for the passage of at least one medical device throughout said corresponding hollow elongate element, in step v), at least the shape of said internal wall of each hollow elongate element is configured to delimit the excursion of said medical device in the corresponding working area according to the local modification to be made in said corresponding bone surface.

15. The method for manufacturing a guiding ancillary according to claims 13 or 14, **characterised in that** the ancillary is made by additive manufacturing.
